Europäisches Patentamt

European Patent Office

Office européen des brevets

(19)

(11) Veröffentlichungsnummer : **0 077 334**
**B1**

(12) **EUROPÄISCHE PATENTSCHRIFT**

(45) Veröffentlichungstag der Patentschrift :
28.11.84

(21) Anmeldenummer : 82900949.7

(22) Anmeldetag : 23.03.82

(86) Internationale Anmeldenummer :
PCT/EP 82/00058

(87) Internationale Veröffentlichungsnummer :
WO/8203625 (28.10.82 Gazette 82/26)

(51) Int. Cl.³ : **C 07 C 63/36, C 07 C 51/265, C 07 C 50/18, C 07 C 46/04, B 01 J 31/40**

(54) VERFAHREN ZUR OXIDATION VON REAKTIVEN AROMATEN.

(30) Priorität : 22.04.81 DE 3115971
03.03.82 DE 3207572

(43) Veröffentlichungstag der Anmeldung :
27.04.83 Patentblatt 83/17

(45) Bekanntmachung des Hinweises auf die Patenterteilung : 28.11.84 Patentblatt 84/48

(84) Benannte Vertragsstaaten :
AT BE CH DE FR GB LI NL SE

(56) Entgegenhaltungen :
DE-A- 1 940 051
FR-A- 1 549 026
FR-A- 2 078 696
US-A- 3 969 405
Patents Abstracts of Japan, Band 2, Nr. 34, Sekt, C, 8.
März 1978 (JP) siehe Seite 4445
Patents Abstracts of Japan, Band 1, Nr. 157, Sekt. C,
14. Dezember 1977 (JP) siehe Seite 3503
Soviet Inventions Illustrated, Derwent Publications
1980, Woche C43, 3., Dezember 1980

(73) Patentinhaber : **Rütgerswerke Aktiengesellschaft**
**Mainzer Landstrasse 217**
**D-6000 Frankfurt a. Main 1 (DE)**

(72) Erfinder : **KNIPS, Ulrich**
**Werver Mark 174**
**D-4618 Kamen-Heeren-Werve (DE)**
Erfinder : **BÖHMER, Bertram**
**Mozartstrasse 8**
**D-4670 Lünen (DE)**
Erfinder : **HERZBERG, Roland**
**Gemeindeplatz 3**
**D-4620 Castrop-Rauxel (DE)**

0 077 334

**Beschreibung**

Die Erfindung betrifft ein Verfahren zur Oxidation von reaktiven Aromaten mit molekularem Sauerstoff in Gegenwart eines Katalysators bestehend aus einer Kobaltverbindung in Essigsäure mit Zusatz einer als Co-Katalysator geeigneten Mangan- und einer Bromverbindung.

Reaktive Aromaten sind solche aromatische Verbindungen, die eine oder mehrere reaktive Kohlenstoff-Wasserstoff-Bindungen haben. Diese können entweder in Methylgruppen eines methylsubstituierten Aromaten sein, oder aber in einem aromatischen Kern. Beispiele für methylsubstituierte reaktive Aromaten sind Toluol, Xylole, Monoalkylnaphthaline wie z. B. 1-Methyl-naphthalin, oder 2-Methylnaphthalin, Dimethylnaphthaline wie z. B. 1,2-Dimethylnaphthalin, 1,3-Dimethylnaphthalin, 1,4-Dimethylnaphthalin, 1,5-Dimethylnaphthalin, 1,6-Dimethylnaphthalin, 1,7-Dimethylnaphthalin, 1,8-Dimethylnaphthalin, 2,3-Dimethylnaphthalin, 2,6-Dimethylnaphthalin oder 2,7-Dimethylnaphthalin, Trimethylnaphthaline oder auch Methylderivate höher anellierter Aromaten. Diese Produkte werden zu den entsprechenden Carbonsäuren oxidiert.

Reaktive Aromaten mit reaktiven Kohlenstoff-Wasserstoff-Bindungen in einem aromatischen Kern sind Anthracen, Halogen- oder Nitroanthracen oder Fluoren. Diese Verbindungen werden zu entsprechenden Chinonen oder zu Fluorenon oxidiert.

Aus US-PS 3 969 405 ist bekannt, Alkylbenzole mit Hilfe von Cobalt- oder Mangansalzen in Gegenwart von Sauerstoff in essigsaurer Lösung zu den entsprechenden aromatischen Carbonsäuren zu oxidieren. Die dabei gebildeten $Co^{2+}$- bzw. $Mn^{2+}$-Ionen werden mittels anodischer Oxidation, Ozon, Peroxiden oder einem Aldehyd wieder zu dreiwertigen Ionen oxidiert und so reaktiviert.

Ähnlich wird gemäß FR-PS 1 549 026 p-Xylol mit Hilfe einer katalytisch wirkenden Cobaltsalzlösung in Essigsäure zu Terephthalsäure oxidiert. Das Cobalt-(II)-salz wird dazu vor der Reaktion mit Peressigsäure in das Cobalt-(III)-salz oxidiert und so aktiviert. Derartige Aktivierungen sind offenbar notwendig, wenn nur einfache Metallsalzkatalysatortypen eingesetzt werden, während sie bei Einsatz von Mischsalzkatalysatoren, wie sie aus DE-OS 2 107 357 bekannt sind, nicht benötigt werden.

Nach DE-OS 2 107 357 erfolgt die Oxidation von Monomethyl- oder Dimethylnaphthalinen mit molekularem Sauerstoff in wäßriger, essigsaurer Lösung unter Verwendung eines Katalysators, der aus einer Kobalt-, einer Mangan- und einer Bromverbindung zusammengesetzt ist.

Nach erfolgter Umsetzung wird ein Teil der Essigsäure abdestilliert, der Rückstand mit viel Wasser vermischt, ausgekocht und das Gemisch abgefühlt und die hergestellte Naphthalincarbonsäure ausgefällt und abgetrennt. Die wäßrige Lösung mit dem Katalysator wird verworfen. Das Verfahren ist arbeitsintensiv und energieaufwendig. Außerdem ist es unwirtschaftlich, nach jedem Reaktionszyklus den Katalysator zu verwerfen. In der DE-OS 2 107 375 wird zwar auch die Möglichkeit angedeutet, nicht umgesetztes Ausgangsmaterial, Zwischenoxidationsprodukt und Katalysator « durch Entfernen von Wasser » zurückzugewinnen und « durch Unterwerfen der verbleibenden Mutterlauge erneut an eine Oxidationsbehandlung im Kreislauf zu führen ». Beachtet man allerdings die Energie, die zum Verdampfen des Wassers notwendig ist, so ist auch diese Verfahrensführung wirtschaftlich nicht vertretbar. Außerdem wurde gefunden, daß die so wiedereingesetzte Katalysatorlösung nahezu ihre gesamte Aktivität verloren hat, so daß ein zweiter Reaktionszyklus mit dem gleichen Katalysator keine nennenswerte Umsetzung mehr ergibt.

Die DE-AS 1 940 051 beschreibt ein Verfahren zur selektiven Oxidation von Anthracenfraktionen mit molekularem Sauerstoff. Die Oxidation erfolgt in carbonsaurer Lösung unter Verwendung eines Katalysators, der aus einer Kobalt-, einer Mangan- und einer Bromverbindung zusammengesetzt ist. Es werden keine Angaben zur Aufarbeitung des Katalysatorsystems gemacht, sondern mit Hinweis auf seinen geringen Preis wird der totale Verlust in Kauf genommen.

Aus Patent Abstracts of Japan, Nr. 34, Band 2, Sekt. C (1978), S. 4445 (JP-A 52 13 39 42) ist bekannt, p-Xylol mit Sauerstoff in Gegenwart eines Katalysators zu oxidieren. Um danach den Katalysator von inhibierenden Phenolen zu befreien und ihn so zu reaktivieren, wird die nach dem Oxidationsschritt erhaltene Reaktionslösung filtriert und destilliert. Der im Destillationsrückstand verbleibende Katalysator wird mit Peroxiden versetzt und mit Wasser aufgeschlämmt. Danach wird die Aufschlämmung separiert und die dabei erhaltene wäßrige Lösung einer weiteren Destillation unterworfen. Dies Verfahren ist extrem arbeits- und energieintensiv und damit nicht wirtschaftlich.

Nach Patent Abstracts of Japan Nr. 157, Band 1, Sec. C (1977), S. 3503 (JP-A 52-101 687) wird p-Xylol in essigsaurer Lösung mit Sauerstoff in Gegenwart eines Co-Mn-Br-Katalysators oxidiert. Um danach den Katalysator wieder zu regenerieren, wird aus der Mutterlauge das Reaktionswasser abdestilliert und die verbleibende Essigsäurelösung unter Zusatz von 1 bis 10 Mol p-Xylol pro g-Atom Katalysatormetall bei Temperaturen im Bereich von 195 bis 260 °C mit Sauerstoff verblasen. Das Verfahren ist apparativ äußerst aufwendig und führt zudem zu einer erheblichen Belastung der Abluft.

Es bestand daher die Aufgabe, ein einfacheres und wirtschaftlicheres Verfahren zur Oxidation von reaktiven Aromaten zu finden, das es ohne großen Aufwand erlaubt, auf die destillative Abtrennung größerer Lösungsmittelmengen zu verzichten und die Katalysatorlösungen mehrfach einsetzen zu können, ohne daß ein beeinträchtigender Aktivitätsverlust auftritt.

Die Lösung der Aufgabe liegt in einem Verfahren gemäß dem Anspruch.

2

0 077 334

Es wurde gefunden, daß der Grund für die Blockierung des Katalysatorsystems darin liegt, daß bei der Oxidation der Aromaten infolge von Nebenreaktionen oder Reaktionen von Begleitern Verbindungen gebildet werden, die die Entstehung stabiler $Co^{3+}$-Komplexe bewirken. Bekanntlich sind Kobalt(III)-Komplexe stabiler als einfache Kobalt(III)-Salze. Das heißt : in diesen Komplexen wird die Oxidationsstufe 3 des Kobalts so fixiert, daß ein Übergang in den zweiwertigen Zustand bei dem vorliegenden carbonsauren Reaktionsmedium verhindert wird. Dadurch wird das Kobaltsalz als Oxidationskatalysator unwirksam.

Behandelt man nun den so ganz oder teilweise blockierten Katalysator mit Kaliumpermanganat, so werden offensichtlich die komplexierenden Nebenprodukte so weitgehend oxidativ abgebaut, daß wieder einfache Kobalt(III)-Salze entstehen. Bei Zugabe einer neuen Menge von reaktiven Aromaten kann ein neuer Reaktionszyklus erfolgen. Diese Reaktionsfolge kann mindestens 10 mal wiederholt werden, bevor eine endgültige Blockierung des Katalysators durch Inaktivierung infolge der Bildung von harzartigen Nebenprodukten eintritt oder die zunehmende Viskositätserhöhung des Reaktionsgemisches durch Anreicherung von Begleitern der reaktiven Aromaten die Gewinnung der kristallisierten Carbonsäuren oder Chinone behindert und im Extremfall unmöglich macht.

Die reaktiven Aromaten können sowohl als Einzelsubstanz als auch in beliebigen Abmischungen miteinander eingesetzt werden. An ihre Reinheit werden keine besonderen Anforderungen gestellt, d. h. die im etwa gleichen Temperaturbereich siedenden verwandten, nicht reaktiven Verbindungen, wie etwa Naphthalin, Diphenyl, Phenanthren oder Carbazol, stören die Oxidationsreaktion nicht. Desgleichen ist eine Entfernung von Schwefelkörpern, wie z. B. des Methylthionaphthens oder Thionaphthens nicht erforderlich. Die Oxidation der reaktiven Aromaten wird in einer Essigsäurelösung durchgeführt, wobei pro Gewichtsteil an reaktiven Aromaten wenigstens 2,5 Gew.-Teile Essigsäure verwendet werden. Die Essigsäure kann geringe Mengen an Wasser enthalten, denn dies verbessert die Löslichkeit der als Katalysator eingesetzten Salze. Andererseits sinkt die Ausbeute an Endprodukt mit steigendem Wassergehalt der Reaktionslösung. Es ist daher die Wassermenge ausreichend, die eingebracht wird, wenn man die als Katalysator dienenden Salze mit ihrem natürlichen Kristallwassergehalt einsetzt. Außerdem wird bei der Oxidationsreaktion Wasser gebildet, das die Essigsäure verdünnt.

Dieses Wasser wird vor dem jeweils folgenden Reaktionszyklus destillativ entfernt.

Als Katalysator für die Oxidationsreaktion dient eine Kombination von Verbindungen, die die drei Komponenten eine Kobaltverbindung (Komponente A), eine Manganverbindung (Komponente B) und Brom oder eine Bromverbindung (Komponente C) in bestimmten Anteilen enthält.

Die anteilmäßigen Mengen der einzelnen Komponenten A, B und C liegen in folgenden Bereichen :

(1)  $1,0 \leq X + Y + Z \leq 10,0$
(2)  $0,1 \leq Z/(X + Y) \leq 2,5$ und
(3)  $0,2 \leq X/Y \leq 20$

Hierin bezeichnet X die Menge des in der genannten Kobaltverbindung enthaltenden Kobalts in Angaben von Gewichtsteile je 50 Gewichtsteile an reaktiven Aromaten, Y bezeichnet die Menge des in der Manganverbindung enthaltenden Mangans in Angaben von Gewichtsteilen je 50 Gew.-Teile reaktiver Aromaten und Z die Menge an Brom oder des in der Bromverbindung enthaltenen Broms in Angaben von Gewichtsteilen je 50 Gewichtsteile reaktiver Aromaten.

Beispiele für geeignete Kobalt- (Komponente A) und Manganverbindungen (Komponente B), die in dem Katalysator gemäß der Erfindung verwendet werden können, sind Kobalt- und Mangansalze von aliphatischen Carbonsäuren mit 1 bis 4 Kohlenstoffatomen, z. B. Ameisen-, Essig-, Propion-, Butter-, Bernsteinsäure, Kobalt- und Mangansalze von aromatischen Carbonsäuren, z. B. Benzoesäure, Phthalsäure, Naphthalinmonocarbonsäure oder Naphthalindicarbonsäure und anorganische Salze von Kobalt und Mangan, z. B. Oxide, Carbonate, basische Carbonate, Chloride und Bromide.

Bevorzugte Salze sind Kobalt(II)- und Mangan(II)-acetat und -bromid. Die Verwendung eines Kobalt- oder Manganbromids hat den Vorteil, daß damit gleichzeitig die Komponente C des Katalysators eingebracht ist. Wenn aber nur Kobaltbromid und Manganbromid als Komponenten (A), (B) und (C) verwendet werden, erfüllt das proportionale Verhältnis zwischen Kobalt, Mangan und Brom, das dem Reaktionssystem zugeführt werden soll, nicht die Bedingung, die durch die vorstehend angegebene Formel (2) vorgeschrieben ist. Demgemäß ist es notwendig, geeignete Mengen von anderen Verbindungen von Kobalt und/oder Mangan, außer Kobalt- und Manganbromid (z. B. Kobalt- und Manganacetat) zusammen mit Kobalt- und/oder Manganbromid zu verwenden und das proportionale Verhältnis unter diesen Verbindungen so einzustellen, daß X, Y und Z die von den Formeln (1), (2) und (3) vorgeschriebenen Bedingungen erfüllen.

Anstelle von Kobalt- oder Manganbromid kann auch Brom oder eine andere Bromverbindung als Komponente C des Katalysators eingesetzt werden, wie andere Metallbromide, Ammoniumbromid, Bromwasserstoff oder organische Bromverbindungen wie z. B. Bromessigsäure oder Benzylbromid.

Die Oxidation der Aromaten erfolgt durch Sauerstoff. Eingesetzt wird molekularer Sauerstoff, entweder als reiner Sauerstoff oder im Gemisch mit einem oder mehreren gegenüber der Reaktion inerten Gasen. Ein derartiges Gemisch ist Luft.

Es ist notwendig, die Oxidation der reaktiven Aromaten unter Druck durchzuführen, und zwar in der

3

Weise, daß der Partialdruck des Sauerstoffs wenigstens 0,5 bar beträgt. Ist der Partialdruck von Sauerstoff niedriger als 0,5 bar, wird die Ausbeute der gewünschten Oxidationsprodukte verringert. Der bevorzugte Partialdruck von Sauerstoff liegt innerhalb des Bereichs von 1 bis 8 bar, insbesondere 2 bis 8 bar. Bei einem Sauerstoffpartialdruck von mehr als 10 bar erhält man zwar auch Oxidationsprodukte, jedoch wird die Ausbeute durch Erhöhen des Sauerstoffpartialdruckes über die vorstehend angegebene obere Grenze hinaus nicht nennenswert gesteigert, so daß eine weitere Erhöhung des Drucks wirtschaftlich nicht sinnvoll ist. Somit erlaubt dieses Verfahren z. B. die Herstellung von Anthrachinon unter wesentlich milderem Druck, als in DE-AS 1 940 051 unter den in den Beispielen belegten optimalen Versuchsbedingungen.

Um eine wirtschaftlich ausreichende Reaktionsgeschwindigkeit zu erreichen, ist es notwendig, die Oxidation des Anthracens bei einer Temperatur oberhalb von 70 °C durchzuführen. Die Reaktionstemperatur sollte andererseits 170 °C nicht übersteigen, da sonst der Anteil der entstehenden unerwünschten Nebenprodukte zu groß wird.

Demnach erfolgt die Oxidation der reaktiven Aromaten, indem diese, Essigsäure und die Katalysator-Kombination in ein Druckgefäß eingebracht und erhitzt werden. Vor oder nach dem Erhitzen wird Sauerstoff oder ein Sauerstoff enthaltendes Gas in das Reaktionsgefäß eingeblasen und der gewählte Druck während der gesamten Reaktionsdauer aufrechterhalten.

Zweckmäßigerweise setzt man die Reaktionsmischungen so zusammen, daß man pro Reaktionszyklus gerade noch eine vollständige Umsetzung des eingesetzten reaktiven Aromaten erzielt, bevor des Katalysator blockiert ist. Das läßt sich erreichen, wenn die Katalysatormischung in einer Menge von 10-20 Gew.-% des eingesetzten Aromaten verwendet wird. Die Reaktion ist beendet, wenn kein Sauerstoff mehr aufgenommen wird.

Nach diesem ersten Reaktionszyklus erfolgt eine Nachbehandlung des Reaktionsgemisches mit Kaliumpermanganat. Hierbei werden die inhibierenden Nebenprodukte so weitgehend oxidativ abgebaut, daß nach Zugabe von frischem reaktivem Aromat ein neuer Reaktionszyklus erfolgen kann.

Kaliumpermanganat besitzt den weiteren Vorteil, daß außer nicht störenden Kaliumionen keine weiteren Fremdionen in das Reaktionsgemisch geraten, da das Mangan bereits Bestandteil des Katalysators ist. Es dient gleichzeitig dazu, Katalysatorverluste auszugleichen.

Die Regenerierung des Katalysators erfolgt zweckmäßigerweise bei erhöhter Temperatur und kann vorteilhaft mit der Entfernung des Reaktionswassers verbunden werden, indem gleichzeitig ein Essigsäure/-Wasser- oder — nach Zugabe von Benzol — ein Benzol/-Wasser-Azeotrop destillativ abgetrennt wird.

Danach ist der Katalysator einsatzbereit zum 2. Reaktionszyklus. Mindestens 5-10 solcher Zyklen können mit einem Katalysatoransatz durchgeführt werden, bevor eine endgültige Blockierung des Katalysators durch Inaktivierung infolge von Verharzung eintritt. Die Anzahl der durchführbaren Reaktionszyklen hängt wesentlich vom Umfang der Viskositätszunahme des Reaktionsgemisches ab. Dabei stellt sich heraus, daß Gemische der reaktiven Aromaten naturgemäß eine häufigere Aufarbeitung bedingen als die reinen Aromaten, wo Nebenreaktionen der Begleiter in geringerem Umfang auftreten.

Die Löslichkeit der Oxidationsprodukte ist im abgekühlten Reaktionsgemisch so gering, daß nach jedem Reaktionszyklus praktisch die gesamte Menge der gebildeten Carbonsäuren oder Chinone auskristallisiert. Restmengen stören den Reaktionsablauf nicht und sind gegen Kochen mit $KMnO_4$ unter den gegebenen Bedingungen weitgehend beständig. Dies bringt den zusätzlichen Vorteil des Verfahrens, daß der aktivierte Katalysator ohne weitere Reinigung zurückgeführt werden kann und daß damit eine einfache diskontinuierliche oder kontinuierliche Verfahrensführung ermöglicht wird.

Wenn nach Durchlaufen des letzten Reaktionszyklus beim Abkühlen die Kristallisation des Oxidationsproduktes sehr erschwert ist, wird zweckmäßig mit Wasser versetzt, worauf das noch gelöste Oxidationsprodukt mit den akkumulierten Begleitstoffen amorph oder mikrokristallin ausfällt. Vorher kann ggf. die Essigsäure weitgehend abdestilliert werden. Aus der wäßrigen Lösung lassen sich die Katalysatorsalze zurückgewinnen. Der zweckmäßige Verfahrensablauf wird aus den Beispielen deutlich.

Beispiele

Beispiel 1

In eine beheizbare und mit einem Rührwerk versehene Druckapparatur mit 0,2 l Inhalt werden 10 g 2-Methylnaphthalin und eine Lösung aus 50 g Essigsäure, 1,5 g $Co(CH_3COO)_2 \cdot 4 H_2O$ und 0,5 g $MnBr_2 \cdot 4 H_2O$ eingebracht. Danach wird das Reaktionsgefäß verschlossen, mit 2 bar Sauerstoff begast und auf 130 °C erhitzt. Über ein Ausgleichsventil wird während dieses Vorgangs der Druck auf 2 bar reguliert. Nach 1 1/2-stündiger Reaktionszeit wird auf etwa 80 °C abgekühlt und entspannt.

Danach werden 0,5 g festes $KMnO_4$ und 15 ml Eisessig zugegeben und damit das Reaktionswasser azeotrop abdestilliert. Es wird abgebrochen, wenn bei langsamer Destillation 15 ml übergegangen sind.

Zum 2. Reaktionszyklus werden erneut 10 g 2-Methylnaphthalin zugegeben, mit 2 bar Sauerstoff begast und 2 1/2 h lang auf 130 °C erhitzt. Anschließend wird wie im Reaktionszyklus 1 der Katalysator mit Hilfe von 0,5 g $KMnO^4$ regeneriert und die Reaktionslösung erneut eingesetzt.

Nach dem dritten Reaktionszyklus wird die Mischung nach der Druckbegasung auf Raumtemperatur

4

abgekühlt, die Kristallmasse abgenutscht und die Lösung nach Zugabe von 0,5 g Co(CH³COO)$_2$ · 4H$_2$O der üblichen Entwässerung und Katalysatorregenerierung unterzogen.

Insgesamt werden mit dem gleichen Katalysataoransatz fünf Reaktionszyklen durchgeführt. Danach wird das Reaktionsgemisch abgekühlt und die ausgefallene Naphthösäure abfiltriert. Das Filtrat wird mit 500 ml kaltem Wasser versetzt und auch diese ausgefallene Naphthösäure abfiltriert, mit reichlich Wasser gewaschen und getrocknet.

Ausbeute : 46,2 g Naphthösäure-(2)

Reinheit : 85 %

entsprechend 65 % der Theorie.

Beispiel 2

Gemäß Beispiel 1 werden in einer 1 l-Apparatur 100 g 1-Methylnaphthalin in 250 g Essigsäure, die 25 g Co(CH$_3$COO)$_2$ · 4H$_2$O und 2,5 g MnBr$_2$ · 4H$_2$O gelöst enthält, innerhalb von 6 Stunden bei 125 °C mit 2 bar Sauerstoff begast. Es wird entspannt und auf Raumtemperatur abgekühlt. Der sich dabei abscheidende Kristallbrei von Rohnaphtösäure wird abgesaugt.

Nach Versetzen der Reaktionslösung mit 2,5 g festem KMnO$_4$ wird auf 100 °C erhitzt und das Reaktionswasser mit wenig Essigsäure (insgesamt 20 g) abdestilliert. Die so erhaltene Lösung wird erneut mit 100 g Methylnaphthalin und Essigsäure (zum Auffüllen der bei der Entwässerung abgetrennten Menge) versetzt und der Sauerstoffbegasung unterworfen.

Nach insgesamt 5 Reaktionszyklen wird der größte Teil der Essigsäure abdestilliert und der Rückstand zur Rückgewinnung der Katalysatorsalze mit Wasser extrahiert. Die im unlöslichen Teil verbleibende Naphthösäure kann mit der verdünnter NaOH-Lösung extrahiert werden.

Über alle 5 Reaktionszyklen können 500 g Rohsäure einer 90 %igen Reinheit gewonnen werden, was einer Ausbeute von 74 % entspricht.

Beispiel 3

Bei analoger Arbeitsweise wie Beispiel 2 werden in 5 Reaktionszyklen 50 g eines 95 %igen 2,6-Dimethylnaphthalinmaterials der Oxidation unterworfen. Es werden jeweils 10 g des Aromaten in einem Gemisch, bestehend aus 50 g Eisessig, 2,5 g Co(CH$_3$COO)$_2$ · 4H$_2$O und O,5 g MnBr$_2$ · 4H$_2$O bei 125 °C und 4 bar O$_2$-Druck zur Reaktion gebracht. Die Reaktivierung des Katalysators erfolgt mit jeweils 0,5 g KMnO$_4$.

Beispiel 4

Analog zu Beispiel 1 werden in einer Apparatur mit 1 l Inhalt 50 g Anthracen (95 %ig) und eine Lösung aus 150 g Essigsäure, 7,5 g Co(CH$_3$COO)$_2$ · 4H$_2$O und 1,3 g MnBr$_2$ · 4H$_2$O eingebracht. Danach wird das Reaktionsgefäß verschlossen, mit 2 bar Sauerstoff begast und auf 130 °C erhitzt. Über ein Ausgleichsventil wird während dieses Vorgangs der Druck auf 4 bar reguliert. Nach 5-6-stündiger Reaktionszeit wird auf etwa 90 °C abgekühlt und entspannt.

Danach werden 1 g festes KMnO$_4$ und 25 ml Eisessig zugegeben und damit das Reaktionswasser abdestilliert. Es wird abgebrochen, wenn bei langsamer Destillation etwa 25 ml übergegangen sind. Danach wird die Mischung auf Raumtemperatur abgekühlt, die Kristallmasse abgenutscht und die Lösung nach Zugabe von 50 g Anthracen erneut der Druckbegasung unterworfen.

Insgesamt wurden mit dem gleichen Katalysatoransatz elf Reaktionszyklen durchgeführt, deren Ergebnisse in der folgenden Tabelle zusammengefaßt sind.

| Nr. des Reaktionszyklus | Einsatz an Anthracen [g] | Ausbeute an Anthrachinon (ber. auf 100%-ige Ware) [g] | [%d. Th.] | Gehalt an Phenanthren [%] |
|---|---|---|---|---|
| 1 | 50 | 38,3 | 68,9 | --- |
| 2 | 50 | 45,3 | 81,6 | 0,3 |
| 3 | 50 | 44,3 | 79,8 | 0,6 |
| 4 | 50 | 39,1 | 70,4 | 0,7 |
| 5 | 50 | 48,1 | 86,6 | 0,5 |
| 6 | 50 | 46,2 | 83,3 | 1,2 |
| 7 | 50 | 47,9 | 86,3 | 0,9 |
| 8 | 50 | 47,4 | 85,4 | 0,7 |
| 9 | 50 | 47,0 | 84,6 | 0,3 |
| 10 | 50 | 44,2 | 79,6 | 0,5 |
| 11 | 50 | 46,3 | 83,4 | 0,5 |

Wie die Ergebnisse des elften Reaktionszyklus zeigen, ist die Reaktivität des Katalysatorgemisches noch keineswegs erschöpft.

**Anspruch**

Verfahren zur Oxidation der Methylgruppe von methylsubstituierten Aromaten zu den entsprechenden aromatischen Carbonsäuren oder von Anthracen oder Halogen- oder Nitroanthracen zu den entsprechenden Chinonen oder von Fluoren zu Fluorenon mit molekularem Sauerstoff unter Druck, wobei der Partialdruck des Sauerstoffs im Bereich von 0,5 bis 8 bar liegt, und bei einer Temperatur im Bereich oberhalb 70 bis 170 °C in Essigsäurelösung in Gegenwart von 10 bis 20 Gew.-% bezogen auf den eingesetzten Aromaten, eines Katalysators, der (A) eine Kobaltverbindung (B) eine Manganverbindung und (C) Brom oder eine Bromverbindung in folgenden Anteilen enthält :

(1) $1,0 \leq X + Y + Z \leq 10,0$
(2) $0,1 \leq Z/(X + Y) \leq 2,5$ und
(3) $0,2 \leq X/Y \leq 20$

wobei X die Menge des in der genannten Kobaltverbindung enthaltenden Kobalts in Angaben von Gewichtsteilen je 50 Gewichtsteile an reaktiven Aromaten, Y die Menge des in der Manganverbindung enthaltenen Mangans in Agaben von Gewichtsteilen je 50 Gew.-Teile reaktiver Aromaten und Z die Menge an Brom oder des in der Bromverbindung enthaltenen Broms in Angaben von Gewichtsteilen je 50 Gewichtsteile reaktiver Aromaten bezeichnen, dadurch gekennzeichnet, daß nach jedem Reaktionszyklus die Mutterlauge mit 2 bis 5 Gew.%, bezogen auf die Menge des eingesetzten Aromaten, festem Kaliumpermanganat und gegebenenfalls Essigsäure versetzt und auf maximal 100 °C erhitzt und dabei das Reaktionswasser aus dem vorangegangen Reaktionszyklus abdestilliert wird.

**Claim**

Process for the oxidation of the methyl group of methyl substituted aromatic compounds to the corresponding aromatic carboxylic acids or of the anthracene or halogen- or nitroanthracene to the corresponding chinone or of the fluorene to fluorenone with molecular oxygen under pressure in the course of which the partial pressure of the oxygen is in the range of 0.5 to 8 bar and the temperature is in the range of above 70 to 170 °C in a solution of acetic acid in the presence of 10 to 20 percent by weight, based on the amount of the used aromatic compound, of a catalyst which contains (A) a cobalt compound, (B) a manganese compound and (C) bromine or a bromine compound in the following proportions :

(1) $1{,}0 \leq X + Y + Z \leq 10{,}0$
(2) $0{,}1 \leq Z/(X + Y) \leq 2{,}5$ and
(3) $0{,}2 \leq X/Y \leq 20$

whereas X is the amount of cobalt in said cobalt compound, Y is the amount of manganese in the manganese compound and Z is the amount of elementary bromine or bromine of the bromine compound, all amounts in percent by weight based on 50 parts by weight of the reactive aromatic compound, characterized in that after each reaction cycle 2 to 5 percent by weight, based on the amount of the used aromatic compound, of solid potassium permanganate and, if needed, acetic acid are added to the mother liquor and heated up to maximum 100 °C and the reaction water from the preceded reaction cycle is distilled off.

## Revendication

Procédé pour transformer par oxydation du groupe méthyle les produits aromatiques méthyl-substitués en les acides carboxyliques aromatiques correspondants ou pour transformer l'anthracène ou les anthracènes halogénés ou nitrés en les quinones correspondantes ou le fluorène en la fluorénone correspondante, à l'aide d'oxygène moléculaire sous pression, la pression partielle de l'oxygène étant située dans le domaine de 0,5 à 8 bars, et à une température située dans le domaine supérieur à 70 à 170°C, en solution dans l'acide acétique en présence de 10 à 20 % en poids, par rapport au produit aromatique mis en œuvre, d'un catalyseur contenant (A) un composé du cobalt, (B) un composé du manganèse et (C) du brome ou un composé du brome dans les proportions suivantes :

(1) $1{,}0 \leq X + Y + Z \leq 10{,}0$
(2) $0{,}1 \leq Z/(X + Y) \leq 2{,}5$ et
(3) $0{,}2 \leq X/Y \leq 20$

X représentant la quantité de cobalt contenue dans le compos du cobalt en question donnée en parties en poids pour chaque fois 50 parties en poids de produits aromatiques réactifs, Y représentant la quantité de manganèse contenue dans le composé du manganèse donnée en parties en poids pour chaque fois 50 parties en poids de produits aromatiques réactifs et Z représentant la quantité de brome ou du brome contenu dans le composé du brome donnée en parties en poids pour chaque fois 50 parties en poids de produits aromatiques réactifs, caractérisé par le fait qu'après chaque cycle réactionnel, la lessive mère est traitée avec 2 à 5 % en poids, par rapport à la quantité de produits aromatiques mise en œuvre, de permanganate de potassium solide et éventuellement d'acide acétique, la température étant portée à au maximum 100°C et l'eau réactionnelle éliminée du cycle réactionnel précédent par distillation.